# EUROPEAN PATENT APPLICATION

(11) **EP 2 356 986 A1**
(43) Date of publication of application: **17.08.2011**
(21) Application number: 09820302.9
(22) Date of filing: 16.10.2009
(51) Int. Cl.: A61K 31/353, C07D 311/62, A61P 3/00

(54) **COMPOSITION FOR TREATMENT OF METABOLIC SYNDROME**

(30) Priority: 16.10.2008 ES 200802925
(71) Applicant: Universitat Rovira I Virgili, 43003 Tarragona (ES)
(72) Inventor: ARDEVOL GRAU, Ana, María, E-43003 Tarragona (ES); AROLA FERRER, Luis, María, E-43003 Tarragona (ES); BAIGES BLANCO, Isabel, María, E-43003 Tarragona (ES); BLADE SEGARRA, María, Cinta, E-43003 Tarragona (ES); BLAY OLIVE, María, Teresa, E-43003 Tarragona (ES); FERNANDEZ LARREA, Juan, Bautista, E-43003 Tarragona (ES); PUJADAS ANGUIANO, Gerard, E-43003 Tarragona (ES); SALVADO ROVIRA, María, Josepa, E-43003 Tarragona (ES); VALLS FONAYET, Josep, E-43003 Tarragona (ES)
(74) Representative: Carpintero Lopez, Francisco
(86) International application number: PCT/ES2009/070443
(87) International publication number: WO 2010/043749

(57) **Abstract**

The invention relates to a composition comprising a combination of a dimer-gallate and a trimer, useful as a medicament for the treatment and/or prevention of metabolic syndrome or of a pathology associated therewith, wherein the dimer-gallate is a procyanidin B-gallate, differing from B2-gallate, and the trimer is a procyanidin C. It also relates to the use of said composition in the preparation of a food, a food additive or a dietary supplement.

## Description

### Field of the Invention

The present invention relates to a specific combination of procyanidins present in grape seed extracts and useful for the treatment and/or prevention of metabolic syndrome.

### Background of the Invention

Metabolic syndrome is common in the Western world and it is important to control it as it is considered a stage prior to the development of diabetes and cardiovascular diseases. It is said that an individual suffers metabolic syndrome when he presents at least three of the following symptoms: overweight, insulin resistance, dyslipidemia, hyperglycemia, hypertension, endothelial inflammation and dysfunction.

Several healthy properties of grape seed procyanidin extracts have been reported that make them good candidates for the prevention of metabolic syndrome and of pathologies connected therewith. Said extracts have a wide variety of compounds with different structures that are very similar to one another and make it difficult to understand their bioactivity against metabolic syndrome. Nonetheless, it is known that simple structured procyanidins have high antioxidant properties and that the structures with a low molecular weight and high degree of galloylation seem more active as antiproliferative agents. However, until now the compounds present in the extracts responsible for their bioactivity against metabolic syndrome have not been identified.

It would therefore be interesting to identify said compounds in order to be able to develop pharmaceutical compositions or dietary supplements containing them, useful for the treatment and/or prevention of metabolic syndrome and other conditions or pathologies associated therewith. Said conditions related with metabolic syndrome include type II diabetes, dyslipoproteinemia, myocardial infarction, stroke and other arteriosclerotic diseases, as well as risk factors of these diseases, including general insulin resistance, abdominal obesity caused by accumulation of abdominal fat, high serum lipid and glucose levels, hypertension and high diastolic and/or systolic arterial pressure.

Therefore, among other advantages, the administration of these specific compositions would prevent, for example, the antagonistic effects that determined identified or unidentified molecules forming part of the total extract can exert, and on the other hand the efficiency of the identified molecules would be optimized.

### Brief Description of the Drawings

Figure 1 shows a graph depicting the bioactivity of the starting extract and of the combination composition of a dimer-gallate and a trimer.
Figure 2 depicts the mass analysis

### Description of the Invention

In one aspect, the present invention relates to a composition comprising a combination of a dimer-gallate and a trimer, wherein the dimer-gallate is a procyanidin B-gallate, differing from B2-gallate, and has general formula: 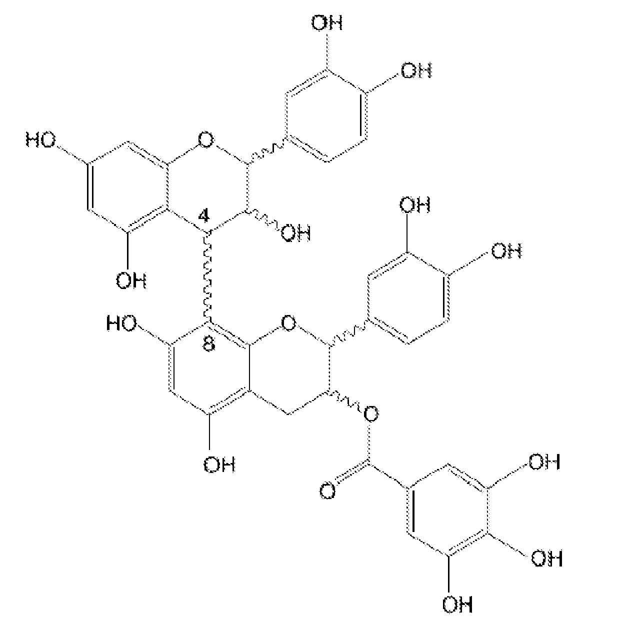 and the trimer is a procyanidin C having general formula: 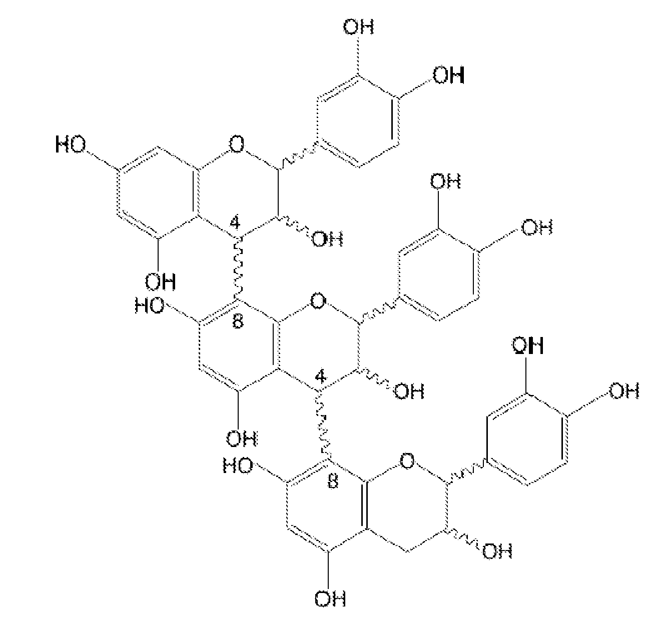 and where the lines with curves indicate α or β stereochemistry

Said composition is obtainable by means of a method comprising the following steps:
- starting from a grape seed extract having a procyanidin content of 76% and a composition of: 1.6% phenolic acids, 20.9% monomers, 20.7% dimers + epigallocatechin-gallate, 17.3% trimers and 39.4% oligomeric forms of 4 or more units;
- fractionating said extract by means of open column low pressure chromatography using silica preconditioned with the acetone:hexane, 65:35, solvent as the stationary phase;
- eluting the flavanol monomers with said solvent,
- establishing a gradient with the acetone:hexane, 80:20, solvent until reaching 100%, and collecting 100 fractions of 15-20 ml each;
- subsequently fractioning the fraction having a retention factor of 0.18 by means of HPLC with the following system: solvents:
   A (formic acid 4.5%); B (acetonitrile:solvent A, 90:10);
   gradient: 0-40% B (0-10 minutes), 40-60% B (10-35 minutes), 60-100% B (35-50 minutes), 100% B (50-60 minutes);
- collecting the elution of the composition comprising the dimer-trimer combination at a retention time of 18.2 minutes,
- evaporating and lyophilizing.

This new composition comprising the dimer-trimer combination is useful in the treatment and/or prevention of metabolic syndrome or of a pathology associated therewith. Therefore, in another aspect the invention relates to said composition as a medicament.

In a particular embodiment, said medicament is suitable for the treatment and/or prevention of metabolic syndrome or of a pathology associated therewith, more particularly said medicament is suitable for the treatment and/or prophylaxis of dyslipidemia.

The invention also relates to the use of a composition comprising said combination of a dimer-gallate and a trimer in the preparation of a medicament for the treatment and/or prevention of metabolic syndrome or of a pathology associated therewith.

The combination composition of a dimer-gallate and a trimer is also useful for the preparation of a food composition or food, a food additive or a dietary supplement. Therefore, in another aspect the invention relates to the use of a composition comprising a combination of a dimer-gallate and a trimer in the preparation of a food, a food additive or a dietary supplement.

In an additional aspect the invention relates to a food, food additive or dietary supplement comprising the combination composition of a dimer-gallate and a trimer. In a particular embodiment, said food or food composition, said food additive or said dietary supplement is characterized by comprising at least an effective amount of the combination composition of a dimer-trimer to produce a therapeutic and/or prophylactic effect in a human being or animal with metabolic syndrome or a pathology associated therewith. In a particular embodiment said associated pathology is dyslipidemia.

In the context of the present invention, a food composition or food relates to a composition containing protein, carbohydrates and/or fat which is used in an organism to sustain growth and vital repair processes, in addition to providing energy. The foods can further contain supplementary substances, such as minerals, vitamins and condiments. The foods in the present invention include foods suitable for human and veterinarian use. The term food includes beverages suitable for human and animal consumption. The term food additive includes direct and indirect additives for foods as defined, for example, by the FDA (21 C.F.R. 170.3(e) (1)). And the term dietary supplement is a product intended for complementing a diet that contains one or more of the following dietary ingredients: a vitamin, a mineral, an herb or botanical element, an amino acid, a dietary substance, a concentrate, a metabolite, an extract or combination of these elements. The mentioned compositions can be prepared in a conventional manner.

The compositions can contain a carrier or excipient chosen according to their use: human or veterinarian, food, additive, dietary or pharmaceutical supplement.

The medicaments provided by the present invention can optionally be administered with at least another active ingredient useful for the treatment of metabolic syndrome and/or other associated pathologies, by oral, buccal, nasal, rectal, intravenous, parenteral, or topical route. The dosage forms can be solids or liquids, such as, for example, capsules, tablets, powders or granules, emulsions, suspensions, pastes, creams, foams, gels, etc., including controlled release forms. The excipients can be determined in each case by a person skilled in the art.

The composition comprising a combination of a dimer-gallate and a trimer can be obtained by means of different purification and/or synthesis methods.

In a particular embodiment, the composition is obtained from a commercial grape seed extract (vitaflavan®). This extract can have certain composition variations according to the batch, but it generically has a procyanidin content of 76% and a composition of: 1.6% phenolic acids, 20.9% monomers, 20.7% dimers + epigallocatechin-gallate (EGCG), 17.3% trimers and 39.4% oligomeric forms of 4 or more units.

According to this particular embodiment, 0.5 grams of the commercial extract are used to start, which are fractionated by means of open column low pressure chromatography. The stationary phase consists of silica (35-70 mesh, Interchim, Monluçon, France) preconditioned with the solvent A (acetone:hexane, 65:35). After eluting the flavanol monomers with this solvent, a gradient is established with solvent B (acetone:hexane, 80:20) until reaching 100%, and a total of 100 fractions of 15-20 ml each are collected with an automatic collector. The content of each fraction is analyzed by thin layer chromatography on PolyGram silica gel 0.2 mm plates with a UV₂₅₄ fluorescent indicator (Macherey-Nagel, Hoerdt, France), with the toluene:acetone:acetic acid mixture (3:3:1, v/v), using the anisaldehyde reagent for development. Based on the thin layer chromatography results, the tubes are grouped into 11 fractions, which also correspond to growing mean degrees of polymerization. Fraction 8 corresponded to a retention factor of Rf=0.18. This fraction 8 is subsequently fractionated by means of semi-preparative HPLC (Varian, model 210) with a 4x250 mm Ulreasep RP18 column (Bischoff, Leonberg, Germany) with the following system: solvents: A (formic acid 4.5%); B (acetonitrile: solvent A, 90:10); gradient: 0-40% B (0-10 minutes), 40-60% B (10-35 minutes), 60-100% B (35-50 minutes), 100% B (50-60 minutes). Detection is monitored at 286 and 306 nm with a UV-vis detector (Varian, model 345). The compounds of interest elute at a retention time of tR=18.2 minutes; they are collected, evaporated and lyophilized. They are stored at -20C.

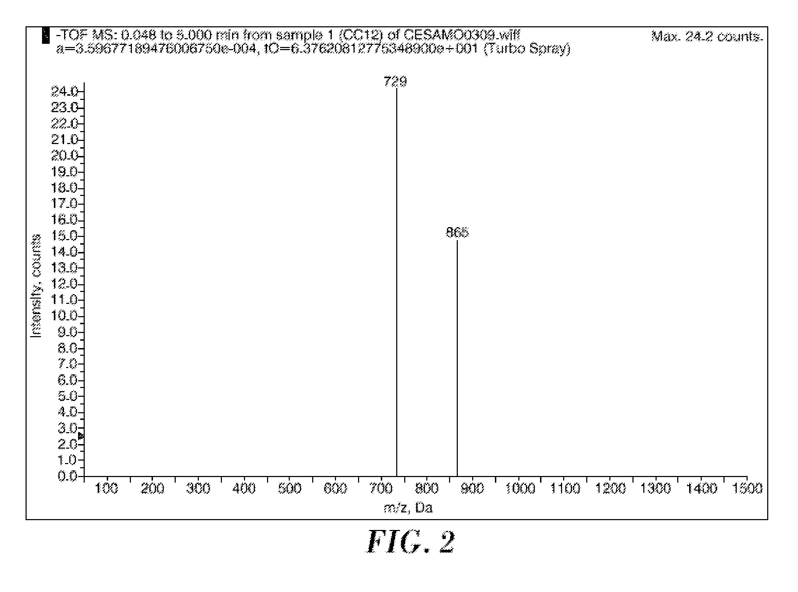

The exact molecular masses of the compounds of interest are determined by means of the HPLC-ESI-MS analysis. Said masses are m/Z= 729 and 865, as described in Example 1, and the spectrum of Figure 2 is shown. The inventors have identified that these molecular weights, corresponding to the structure of a dimer-gallate and of a trimer, respectively, are the components capable of significantly reproducing the previously described effect of the total extract.

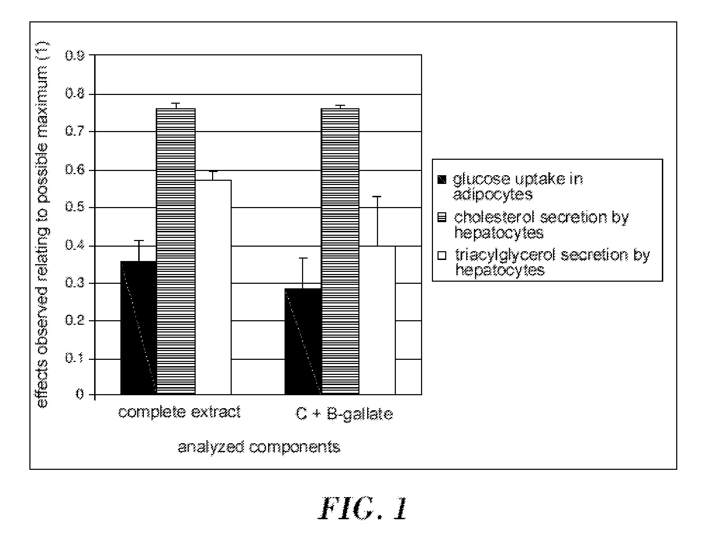

Therefore, the inventors have surprisingly found that said specific combination of a dimer-gallate and of a trimer has metabolic effects because it acts by stimulating glucose uptake in adipocytes and stopping cholesterol secretion by hepatocytes and the secretion of triacylglycerols by hepatocytes (Figure 1). In order to perform these assays, the cell cultures and measurements described in detail in Example 2 were used, and the effect of the combination of dimer-gallate and trimer of the invention on glucose uptake stimulation on 3T3-L1 adipocytes and triglyceride and total cholesterol secretion by HepG2 hepatocytes was studied.

The specific combination of a dimer-gallate and of a trimer reproduces the bioactivity which the total grape seed extract has.

Illustrative examples of the invention are described below in order to better understand the invention and by no means should they be considered as a limitation of the scope thereof.

### EXAMPLES

### Example 1: Characterization of the structures

The determination of the structures present in the composition has been carried out by means of mass spectrometry analysis of the composition, which shows that it is the combination of a trimer (MW= 865) and a dimer-gallate (MW= 729).

The molecular weight was determined in a first approach by means of HPLC-ESI-MS using a Micromass "Platform II" mass spectrometer (Manchester, UK) with electrospray injection (ESI) coupled to a liquid chromatography apparatus. The detection was performed in negative ion mode [M-H]- because the proanthocyanidins readily shield a proton generating negative ions. A low voltage was used to prevent fragmentation and the products were identified by their molecular peaks.

The chromatographic peaks isolated in the semi-preparative HPLC were also characterized by means of Maldi-TOF. The MALDI-MS spectra were obtained with a Micromass TofSpec Maldi-Tof mass spectrometer (Manchester, UK). The instrument is equipped with a pulsed N2 laser emitting at 337 nm with an amplitude of 4 ns, and provided with a delayed ion extraction source. The spectra were recorded in positive mode with a reflectron with an acceleration voltage of 20 kV.

### Example 2

### Cell culture:

3T3-L1 preadipocytes were cultured and induced to differentiate as described in (Ardévol, A. et al., (2000). Changes in lipolysis and hormone-sensitive lipase expression caused by procyanidins in 3T3-L1 adipocytes. Int J Obes, 24, 319-324). Ten days after differentiation, the fully differentiated adipocytes were washed twice with PBS and were incubated at 37°C with DMEM containing 0.2% BSA (fasting medium) for 2 hours. For the last 30 minutes of this treatment, the cells were treated with insulin or the indicated combination of molecules (C+B-gallate).

HepG2 cells (ATCC code HB-8065, Manassas, VA, USA.) are propagated in DMEM (Cambrex) and cultured as described in (Puiggros, F., et al., (2005). Grape Seed Procyanidins Prevent Oxidative Injury by Modulating the Expression of Antioxidant Enzyme Systems. J.Agric.Food Chem., 53(15), 6080-6086). The only modification was the addition of 25 mM HEPES (Sigma) in the culture medium. For the experiments, the HepG2 cells were seeded at 750,000 cells/well (plates of 12), leaving them to grow for two days (80% confluence) in a propagation medium. The medium was replaced 16 hours before treatment.

### Measurements

2-deoxyglucose uptake: the glucose uptake was determined by measuring the 2-deoxy-D-[3H] glucose uptake, as described in (Pinent, M., et al., (2004). Grape seed-derived procyanidins have an antihyperglycemic effect in streptozotocin-induced diabetic rats and insulinomimetic activity in insulin-sensitive cell lines. Endocrinology, 145(11), 4985-4990). Each condition has been performed in triplicate.

Lipid analysis: For the de novo synthesis of triglycerides and cholesterol, the HepG2 cells were seeded in 12 plates. The medium was replaced 16 hours before the treatment. The procyanidins and ¹⁴C-acetate (0.6 µCi/ml) were added to the media and then 6 hours after the treatment the culture media and the cells were collected and the lipids extracted using hexane:isopropanol (3:2). The thin layer chromatography was performed using petroleum ether:diethyl ether:NH3 (40:10:0.1) and with an additional separation using hexane/MTBE/NH3 (30:20:0.1) to obtain fractions of free cholesterol, esterified cholesterol and triacylglycerols. Each fraction was scraped and quantified by liquid scintillation counting. The values were corrected per mg of protein determined according to the Bradford methodology (Bradford, M. M. (1976). A rapid and sensitive method for quantitation of microgram quantities of protein utilizing the principle of protein-dye binding. Anal Biochem, 72, 248-254).

### Calculations and statistical analysis:

The results are expressed as the mean ± SEM. The effects were evaluated by means of the T-test. All the calculations were made using the SPSS software.

## Claims

1. Composition comprising a combination of a dimer-gallate and a trimer, wherein the dimer-gallate is a procyanidin B-gallate, differing from B2-gallate, and has general formula: 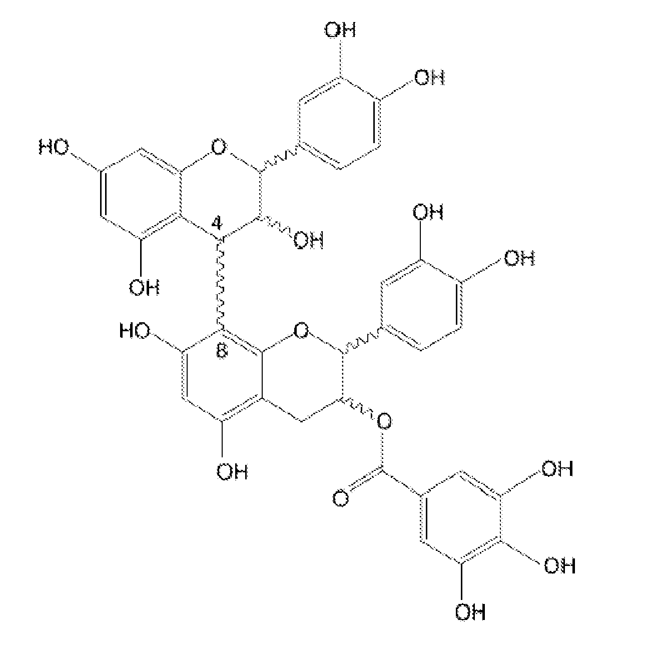 and the trimer is a procyanidin C having general formula: 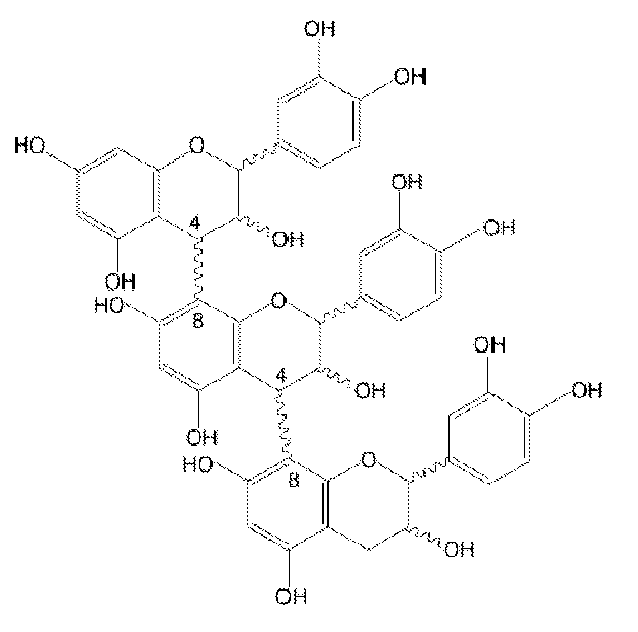 and where the lines with curves indicate α or β stereochemistry obtainable by means of a method comprising the following steps:
- starting from a grape seed extract having a procyanidin content of 76% and a composition of: 1.6% phenolic acids, 20.9% monomers, 20.7% dimers + epigallocatechin-gallate, 17.3% trimers and 39.4% oligomeric forms of 4 or more units;
- fractionating said extract by means of open column low pressure chromatography using silica preconditioned with the acetone:hexane, 65:35, solvent as the stationary phase;
- eluting the flavanol monomers with said solvent,
- establishing a gradient with the acetone:hexane, 80:20, solvent until reaching 100%, and collecting 100 fractions of 15-20 ml each;
- subsequently fractioning the fraction having a retention factor of 0.18 by means of HPLC with the following system: solvents:
Solvent A: formic acid 4.5%
Solvent B: acetonitrile: solvent A at a relative proportion of 90:10;
gradient: 0-40% B (0-10 minutes), 40-60% B (10-35 minutes), 60-100% B (35-50 minutes), 100% B (50-60 minutes);
- collecting the elution of the composition comprising the dimer-trimer combination at a retention time of 18.2 minutes,
- evaporating and lyophilizing.

2. Composition comprising a combination of a dimer-gallate and a trimer, wherein the dimer-gallate is a procyanidin B-gallate, differing from B2-gallate, according to claim 1 as a medicament.

3. Composition according to claim 2 as a medicament for the treatment and/or prevention of metabolic syndrome or of a pathology associated therewith.

4. Composition according to claim 3 as a medicament for the treatment and/or prophylaxis of dyslipidemia.

5. Use of a composition according to claim 1, comprising a combination of a dimer-gallate and a trimer, in the preparation of a medicament for the treatment and/or prevention of metabolic syndrome or of a pathology associated therewith.

6. Use of a composition according to claim 1, in the preparation of a food, a food additive or a dietary supplement.

7. Food, food additive or dietary supplement comprising an effective amount of a combination of a dimer-gallate and a trimer according to claim 1, to produce a therapeutic and/or prophylactic effect in a human being or animal with metabolic syndrome or a pathology associated therewith.

8. Food, food additive or dietary supplement according to claim 7, wherein the associated pathology is dyslipidemia.
